# EUROPÄISCHE PATENTSCHRIFT

(11) **EP 2 611 814 B1**
(45) Veröffentlichungstag und Bekanntmachung des Hinweises auf die Patenterteilung: **24.09.2014**
(21) Anmeldenummer: 11748433.7
(22) Anmeldetag: 29.08.2011
(51) Int. Cl.: C07D 495/14

(54) **VERFAHREN ZUR HERSTELLUNG VON DITHIIN-TETRACARBOXIMIDEN**
METHOD FOR PRODUCING DITHIIN-TETRACARBOXIMIDES
PROCÉDÉ DE PRODUCTION DE DITHIINE-TÉTRACARBOXIMIDES

(30) Priorität: 09.09.2010 US 381163 P; 03.09.2010 EP 10175181
(43) Veröffentlichungstag der Anmeldung: 10.07.2013
(73) Patentinhaber: Bayer Intellectual Property GmbH, 40789 Monheim (DE)
(72) Erfinder: HIMMLER, Thomas, 51519 Odenthal (DE); VOLZ, Frank, 50825 Köln (DE); GELLER, Thomas, 51519 Odenthal (DE)
(74) Vertreter: BIP Patents
(86) Internationale Anmeldenummer: PCT/EP2011/064833
(87) Internationale Veröffentlichungsnummer: WO 2012/028588

(56) Entgegenhaltungen:
- VALLA ALAIN ET AL: "Atypical oxidation reaction by thionyl chloride. Easy two-step synthesis of N-alkyl-1,4-dithiines", SYNTHETIC COMMUNICATIONS, TAYLOR & FRANCIS GROUP, PHILADELPHIA, PA LNKD- DOI:10.1080/00397910600943600, Bd. 36, Nr. 23, 1. November 2006 (2006-11-01), Seiten 3591-3597, XP002599895, ISSN: 0039-7911 in der Anmeldung erwähnt
- ZENTZ ET AL.: "Syntheses, in vitro antibacterial and antifungal activities of a series of N-alkyl, 1,4-dithiines", FARMACO, SOCIETA CHIMICA ITALIANA, PAVIA, IT LNKD- DOI:10.1016/J.FARMAC.2005.06.015, Bd. 60, Nr. 11-12, 1. November 2005 (2005-11-01), Seiten 944-947, XP005151567, ISSN: 0014-827X
- F. BRISSE ET AL.: "N,N'-Dimethyl-1,4-dithiine-1,2:4,5-tetrac arboximide and N,N'-dimethyl-1,4-diselenine-1,2:4,5-tetra carboximide", ACTA CRYSTALLOGRAPHICA, SECTION C: CRYSTAL STRUCTURE COMMUNICATIONS, Bd. C56, Nr. 2, 2000, Seiten 190-192, XP002607970, ISSN: 0108-2701
- J. Y. YUN ET AL.: "Quantitative regioselective Diels-Alder reaction of an unsymmetrical 1,4-dithiin and anthracene through heterogeneous solid state conversion", DYES AND PIGMENTS, Bd. 83, Nr. 2, 6. Dezember 2008 (2008-12-06), Seiten 262-265, XP002607971, ISSN: 0143-7298
- K. HAYAKAWA ET AL.: "Reagent design and study of 1,4-dithiins as a promising class of reagents (synthons) for cycloaddition. Diels-Alder reactions with anthracene derivatives via charge-transfer complexes", J. AM. CHEM. SOC., Bd. 104, Nr. 25, 1982, Seiten 7136-7142, XP002607972, ISSN: 0002-7863
- S. GÜLTEN: "The synthesis and characterization of solvatochromic maleimide-fused N-allyl- and N-alkyl-substituted 1,4-dithiines and Diels-Alder reactions with anthracene", J. HETEROCYCLIC CHEM., Bd. 47, Nr. 1, 8. Januar 2010 (2010-01-08) , Seiten 188-193, XP000002659044,

## Beschreibung

Die vorliegende Erfindung betrifft ein neues Verfahren zur Herstellung von Dithiin-tetracarboximiden.

Dithün-tetracarboximide als solche sind bereits bekannt. Ebenso ist bekannt, dass diese Dithiin-tetracarboximide als Anthelminthika gegen innere Parasiten von Tieren, insbesondere Nematoden, verwendet werden können und insektizide Wirkung aufweisen (vgl. US 3,364,229). Außerdem ist bekannt, dass bestimmte Dithiin-tetracarboximide antibakterielle Wirkung besitzen und gegen Erreger von Mykosen beim Menschen eine gewisse Wirkung aufweisen (vgl. II Farmaco 2005, 60, 944-947). Weiterhin ist bekannt, dass Dithiin-tetracarboximide als Pigmente in elektrophotographischen Photorezeptoren oder als Farbstoffe in Lacken und Polymeren eingesetzt werden können (vgl. JP-A 10-251265, PL-B 143804).

Dithiin-tetracarboximide der Formel (I) 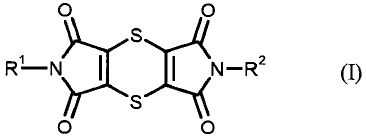 in welcher
- R¹ und R²: gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
- R³: für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
- R⁴: für Hydroxy, C₁-C₄-Alkyl oder C₁-C₄-Alkoxy steht,
können auf verschiedene bekannte Weisen hergestellt werden.

Beispielsweise wird in einem Verfahren (vgl. US 3,364,229; Chem. Ber. 1967, 100, 1559-1570) in einer ersten Stufe Dichlormaleinsäureanhydrid der Formel (II) mit einem Amin der Formel (III) gegebenenfalls in Gegenwart eines Verdünndungsmittels umgesetzt. Anschließend werden dann die so erhaltenen Dichlormaleinsäureimide der Formel (IV) mit einer Schwefelverbindung (z.B. Schwefelwasserstoff oder Thioharnstoff) umgesetzt. Die Herstellung der Dithiin-tetracarboximide der Formel (I) nach diesem Verfahren kann durch das folgende Schema veranschaulicht werden:

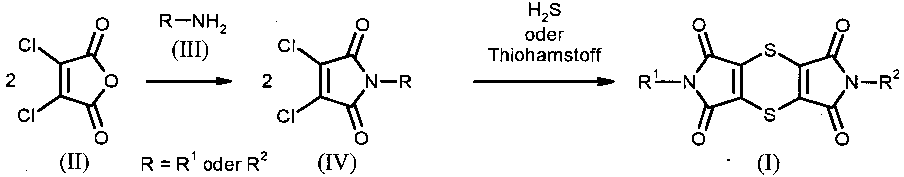

Dieses Verfahren hat den Nachteil, dass beispielsweise der Umgang mit dem hochgiftigen Schwefelwasserstoffgas technisch sehr schwierig und aufwendig ist. Bei der Verwendung von Thioharnstoff werden außer dem Zielprodukt unerwünschte Nebenprodukte erhalten, die nur sehr schwierig zu entfernen sind und die erreichbaren Ausbeuten verschlechtern (vgl. J. Heterocycl. Chem. 1988, 25, 901-906).

In einem weiteren bekannt gewordenen Verfahren (vgl. Synthetic Communications 2006, 36, 3591-3597) wird in einer ersten Stufe Bernsteinsäureanhydrid der Formel (V) mit einem Amin der Formel (III) gegebenenfalls in Gegenwart eines Verdünndungsmittels umgesetzt. Anschließend werden die so erhaltenen Bernsteinsäuremonoamide der Formel (VI) 6 Stunden mit einem großen Überschuss an Thionylchlorid in Gegenwart von Dioxan als Verdünnungsmittel bei Raumtemperatur umgesetzt, wobei in einer Abfolge zahlreicher Reaktionsschritte schließlich die Dithiin-tetracarboximide der Formel (I) erhalten werden. Die Dithiin-tetracarboximide werden wahlweise direkt aus dem Reaktionsgemisch oder nach Versetzen mit Wasser durch Filtration isoliert. Je nach Reaktionsbedingungen (Verdünnungsmittel) und Art der Reste R können unter Umständen die Dithiindiisoimide der Formel (VII) isoliert werden, bevor man sie in die Dithiin-tetracarboximide der Formel (I) überführt. Diese Herstellungsmethode der Dithiin-tetracarboximide der Formel (I) kann durch das folgende Schema veranschaulicht werden:

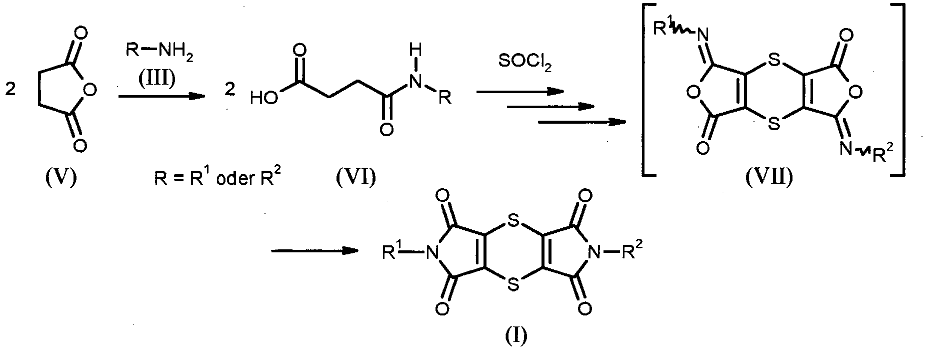

Nachteilig an diesem Verfahren sind die lange Reaktionszeit sowie das Ergebnis, dass entweder die erhaltenen Ausbeuten in der Regel etwa 30-40% der Theorie nicht überschreiten oder aber die Reinheiten der isolierten Produkte ungenügend sind (s. Vergleichsbeispiele). Außerdem ist bei einer wässrigen Aufarbeitung des Reaktionsgemisches nachteilig, dass dabei große Mengen Thionylchlorid vernichtet werden; die entstehenden Gase (SO₂ und HCl) müssen entsorgt werden. Ebenfalls nachteilig ist der Umstand, dass erfahrungsgemäß (s. Vergleichsbeispiele) das Produkt nicht in einer Fraktion erhalten wird. Vielmehr ist es häufig so, dass nach einer ersten Produktisolierung durch Filtration aus dem Filtrat nach längerem Stehen (beispielsweise über Nacht), weiteres Produkt ausfällt, das erneut durch Filtration isoliert werden muss. Zuweilen muss dieser Vorgang nochmals durchgeführt werden. Diese Arbeitsweise ist sehr umständlich und zeitraubend.

Es besteht demnach weiterhin Bedarf an einem technisch einfachen und ökonomischen Herstellverfahren für Dithiin-tetracarboximide der Formel (I).

Es wurde ein neues Verfahren zum Herstellen von Dithiin-tetracarboximide der allgemeinen Formel (I) 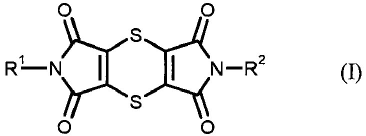 in welcher R¹ und R² die oben angegebenen Bedeutungen haben,
gefunden, dadurch gekennzeichnet, dass man
in einer ersten Stufe Bernsteinsäuremonoamide der Formel (VI) 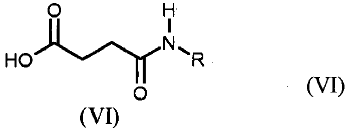 in welcher R für R¹ oder R² steht,
mit einem Überschuss an Thionylchlorid gegebenenfalls in Gegenwart eines Verdünnungsmittels umsetzt,
anschließend den Überschuss an Thionylchlorid entfernt und das so erhaltene Produktgemisch in einer zweiten Stufe in einem Gemisch aus einem organischen Lösungsmittel, Wasser und einem Phasentransferkatalysator in die Dithiin-tetracarboximide der Formel (I) überführt.

Auf diese Weise können die Dithiin-tetracarboximide der Formel (I) in höherer Ausbeute, kürzerer Zeit und besserer Reinheit erhalten werden. Zudem ergibt sich die Möglichkeit zur Rückgewinnung des organischen Lösungsmittels.

Das im ersten Schritt des erfindungsgemäßen Verfahrens erhaltene Produktgemisch enthält auch bereits Dithiin-tetracarboximide der Formel (I), als Hauptkomponenten jedoch Polysulfide der Formel (IX), 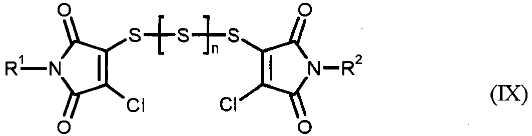 sowie, je nach Aufarbeitungsmethode, auch Thiosulfonsäurederivate der Formel (VIII)

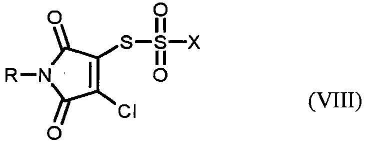

In den Thiosulfonsäurederivaten der allgemeinen Formel (VIII) steht R für die oben angegebenen Bedeutungen von R¹ bzw. R² und X für Chlor oder Hydroxy.

In den Polysulfiden der allgemeinen Formel (IX) stehen R¹ und R² für die oben angegebenen Bedeutungen und n für 0, 1, 2, 3, 4, 5, 6, 7 oder 8.

Verbindungen der allgemeinen Formel (VIII) erhält man neben anderen Produkten dann, wenn das Reaktionsgemisch nach der Umsetzung der Verbindungen der allgemeinen Formel (VI) mit Thionylchlorid eingeengt wird.

Verbindungen der allgemeinen Formel (IX) erhält man neben anderen Produkten dann, wenn man das Reaktionsgemisch nach der Umsetzung der Verbindungen der allgemeinen Formel (VI) mit Thionylchlorid einengt, in einem inerten, nicht mit Wasser mischbaren Lösungsmittel wie beispielsweise Methylenchlorid löst und bei Raumtemperatur mit Wasser ausschüttelt. Nach Abtrennen der organischen Phase, Trocknen und Einengen erhält man ein Gemisch, das neben Dithiin-tetracarboximiden der Formel (I) hauptsächlich Verbindungen der allgemeinen Formel (IX) enthält.

Das erfindungsgemäße Verfahren zur Herstellung der Dithiin-tetracarboximide der Formel (I) kann durch das folgende Schema veranschaulicht werden:

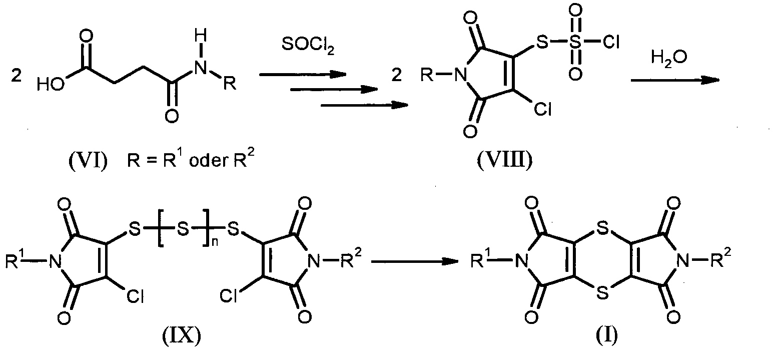

Die bei der Durchführung des erfindungsgemäßen Verfahrens als Ausgangstoffe eingesetzten Bernsteinsäuremonoamide sind durch die Formel (VI) allgemein definiert. R steht für die Bedeutungen von R¹ oder R².
- R¹ und R²: sind bevorzugt gleich oder verschieden und stehen bevorzugt für Wasserstoff, für gegebenenfalls einfach oder mehrfach durch Fluor, Chlor, Brom, -OR³, -COR⁴ substituiertes C₁-C₆-Alkyl, gegebenenfalls einfach oder mehrfach durch Chlor, Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl).
- R¹ und R²: sind besonders bevorzugt gleich oder verschieden und stehen besonders bevorzugt für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-Phenethyl, 2-Phenethyl oder 2-Methyl-2-phenethyl.
- R¹ und R²: sind ganz besonders bevorzugt gleich oder verschieden und stehen ganz besonders bevorzugt für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.
- R¹ und R²: stehen insbesondere bevorzugt gleichzeitig für Methyl.
- R³: steht bevorzugt für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl.
- R³: steht besonders bevorzugt für Wasserstoff, Methyl, Methylcarbonyl oder für Phenyl.
- R⁴: steht bevorzugt für Hydroxy, Methyl, Ethyl, Methoxy oder Ethoxy.
- R⁴: steht besonders bevorzugt für Hydroxy oder Methoxy.

Besonders bevorzugt wird Bernsteinsäuremethylamid als Ausgangsstoff eingesetzt, wodurch man als Endprodukt die Verbindung (I-1) 2,6-Dimethyl-1H,5H-[1,4]dithiino[2,3-c:5,6-c']dipyrrol-1,3,5,7(2H,6H)-tetron erhält.

Setzt man Bernsteinsäuretertiärbutylamid als Ausgangsstoff ein, erhält man die Verbindung (I-2) 2,6-Di-tert-butyl-1H,5H-[1,4]dithiino[2,3-c:5,6-c']dipyrrol-1,3,5,7(2H,6H)-tetron als Endprodukt.

Setzt man Bernsteinsäurecyclohexylamid als Ausgangsstoff ein, erhält man die Verbindung (I-3) 2,6-Dicyclohexyl-1H,5H-[1,4]dithiino[2,3-c:5,6-c']dipyrrol-1,3,5,7(2H,6H)-tetron als Endprodukt.

Setzt man Bernsteinsäurepropylamid als Ausgangsstoff ein, erhält man die Verbindung (I-4) 2,6-Dipropyl-1H,5H-[1,4]dithiino[2,3-c:5,6-c']dipyrrol-1,3,5,7(2H,6H)-tetron als Endprodukt.

Als Zwischenprodukte erhält man besonders bevorzugt
(VIII-1) S-(4-Chlor-1-methyl-2,5-dioxo-2,5-dihydro-1H-pyrrol-3-yl)chlorothiosulfat (R = Me, X = Cl),
(IX-1) 3,3'-Trisulfan-1,3-diylbis(4-chlor-1-methyl-1H-pyrrol-2,5-dion) (R¹ = R² = Me, n = 1)
(IX-2) 3,3'-Disulfanedlylbis(4-chlor-1-methyl-1H-pyrrol-2,5-dion) (R¹ = R² = Me, n = 0)
(IX-3) 3,3'-Disulfanediylbis(1-tert-butyl-4-chlor-1H-pyrrol-2,5-dion) (R¹ = R² = t-Bu , n = 0)
(IX-4) 3,3'-Trisulfan-1,3-diylbis(1-tert-butyl-4-chlor-1H-pyrrol-2,5-dion) (R¹ = R² = t-Bu, n = 1)
(IX-5) 3,3'-Trisulfan-1,3-diylbis(4-chlor-1-cyclohexyl-1H-pyrrol-2,5-dion) (R¹ = R² = Cyclohexyl, n = 1)

Die Menge an Thionylchlorid im ersten Schritt des erfindungsgemäßen Verfahrens liegt zwischen 2 und 100 Mol pro Mol Bernsteinsäuremonoamid der Formel (VI). Bevorzugt verwendet man zwischen 4 und 50 Mol, besonders bevorzugt Mengen zwischen 10 und 40 Mol pro Mol Bernsteinsäuremonoamid der Formel (VI).

Die Reaktionstemperatur im ersten Schritt des erfindungsgemäßen Verfahrens kann in weiten Grenzen variiert werden und liegt zwischen 0°C und 150°C. Zur Erzielung befriedigender Raum-Zeit-Ausbeuten wird man bevorzugt bei Temperaturen zwischen 20°C und 120°C arbeiten; besonders bevorzugt zwischen 30°C und 100°C.

Die Reaktionszeit im ersten Schritt des erfindungsgemäßen Verfahrens liegt zwischen 10 Minuten und 24 Stunden. Bevorzugt arbeitet man zwischen 30 Minuten und 6 Stunden, besonders bevorzugt zwischen 1 und 4 Stunden.

Der erste Schritt des erfindungsgemäßen Verfahrens kann gegebenenfalls in Gegenwart eines unter den Reaktionsbedingungen möglichst inerten Verdünnungsmittels durchgeführt werden. Als solche Verdünnungsmittel seien beispielhaft aliphatische Kohlenwasserstoffe wie Pentan, Hexan, Heptan, Cyclohexan, Methylcyclohexan, Octan, Isooctan, chlorierte Kohlenwasserstoffe wie Methylenchlorid, Chloroform, 1,2-Dichlorethan, aromatische Kohlenwasserstoffe wie Toluol, Xylol, Mesitylen, Ethylbenzol, Anisol, chlorierte aromatische Kohlenwasserstoffe wie Chlorbenzol, Dichlorbenzol, Ether wie Diethylether, Methyl-tert-butylether, Tetrahydrofuran, Dioxan, Nitrile wie Acetonitril, Propionitril, Butyronitril, Ester wie Essigsäuremethylester und Essigsäureethylester genannt. Bevorzugt arbeitet man in Toluol, Xylol, Mesitylen, Ethylbenzol, Chlorbenzol, 1,2-Dichlorbenzol oder ohne Verdünnungsmittel.

Die Entfernung des Thionylchlorids kann prinzipiell durch Hydrolyse mit Wasser erfolgen. Bevorzugt wird das Thionylchlorid durch Abdestillieren unter vermindertem Druck entfernt.

Das gegebenenfalls vorhandene Verdünnungsmittel kann ebenfalls unter vermindertem Druck abdestilliert und, falls gewünscht, durch ein anderes Lösungsmittel ersetzt werden. Bevorzugt wird man jedoch nur das überschüssige Thionylchlorid abdestillieren und dann nach Zugabe von Wasser und Phasentransferkatalysator die Umsetzung im selben Lösungsmittel fortsetzen.

Im zweiten Schritt des erfindungsgemäßen Verfahrens wird der nach Entfernung des überschüssigen Thionylchlorids und gegebenenfalls des Verdünnungsmittels erhaltene Rückstand in einem neuen Verdünnungsmittel gelöst und nach Zugabe eines Phasentransferkatalysators durch Erwärmen in diesem Lösungsmittel in die Dithiin-tetracarboximide der Formel (I) überführt. Bevorzugt wird das Reaktionsgemisch hierbei gerührt

Im zweiten Schritt des erfindungsgemäßen Verfahrens verwendet man organische Lösungsmittel oder Lösungsmittelgemische. Diese Lösungsmittel sind bevorzugt nur geringfügig mit Wasser mischbar.

Als Verdünnungsmittel für den zweiten Schritt des erfindungsgemäßen Verfahrens eignen sich im Einzelnen Kohlenwasserstoffe wie Hexan, Heptan, Cyclohexan, Methylcyclohexan, Octan, Isooctan, Toluol, Xylole, Mesitylen, Ethylbenzol, Chlorbenzol, Dichlorbenzol, Nitrobenzol, Wasser oder Gemische dieser Verdünnungsmittel.

Bevorzugt verwendet man Hexan, Heptan, Cyclohexan, Methylcyclohexan, Octan, Isooctan, Toluol, Xylole, Mesitylen, Chlorbenzol, Dichlorbenzol, Wasser oder Gemische dieser Verdünnungsmittel.

Ganz besonders bevorzugt verwendet man Gemische aus Wasser und Toluol, Xylol oder Chlorbenzol.

Das Mischungsverhältnis Wasser zu organischem Lösungsmittel kann dabei in weiten Grenzen von beispielsweise 9 zu 1 bis 1 zu 9 variiert werden.

Als Phasentransferkatalysatoren (PTC) können prinzipiell alle Verbindungen mit bekannter Wirkung als PTC eingesetzt werden. Bei solchen Verbindungen kann es sich beispielsweise um Phasentransferkatalysatoren aus der Reihe der quaternären Ammoniumsalze oder der quaternären Phosphoniumsalze handeln.

Dieser Phasentransferkatalysator besitzt vorzugsweise die allgemeine Formel (X) 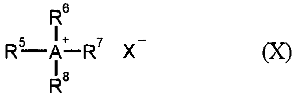 in welcher
- R⁵, R⁶, R⁷ und R⁸: unabhängig voneinander gleich oder verschieden sind und für geradkettiges oder verzweigtes C₁-C₂₈-Alkyl, C₆-C₁₀-Aryl oder Benzyl stehen,
- X: für Halogen, Hydrogensulfat, Sulfat, Dihydrogenphosphat, Hydrogenphosphat, Phosphat oder Acetat (bevorzugt für Brom, Chlor, Fluor, Hydrogensulfat, Sulfat, Phosphat und Acetat) steht,
- A: für N oder P steht.

Als solche Phasentransferkatalysatoren seien beispielhaft Tetrabutylammoniumfluorid, -chlorid, -bromid, -iodid, -acetat, -hydrogensulfat, Tetraethylammoniumbromid, -iodid, Methyltributylammoniumchlorid, -bromid, -iodid, -acetat, -hydrogensulfat, Benzyl-dodecyl-dimethylammoniumchlorid, -bromid, Benzyltriethylammoniumbromid, -chlorid, Dodecyltrimethylammoniumchlorid, -bromid, Tetradecyl-trimethylammoniumchlorid, -bromid, Methyl-trioctylammoniumchlorid, Methyl-tridecylammoniumchlorid, Tetraoctylammoniumbromid, -chlorid, Didecyl-dimethyl-ammoniumchlorid, -bromid, Tetraphenylphosphoniumbromid, Ethyl-triphenylphosphoniumbromid, Ethyl-triphenylphosphoniumiodid und Ethyl-triphenylphosphoniumacetat genannt.

Darüber hinaus können auch Phasentransferkatalysatoren wie 4-Dialkylamino-pyridiniumsalze oder Hexaalkylguanidiniumsalze zur Anwendung kommen.

Bevorzugt werden Methyl-trioctylammoniumchlorid (Handelsname Aliquat^{®} 336; liegt in Mischung mit Methyl-tridecylammoniumchlorid vor), Methyl-tridecylammoniumchlorid, -bromid, Tetraoctylammoniumbromid, -chlorid, Dodecyltrimethylammoniumchlorid, -bromid, Tetradecyl-trimethylammoniumchlorid, -bromid, Didecyl-dimethyl-ammoniumchlorid, -bromid und Benzyl-dodecyl-dimethylammonium- chlorid oder -bromid als Phasentransferkatalysatoren verwendet.

Die Menge an Phasentransferkatalysator im erfindungsgemäßen Verfahren kann in weiten Grenzen variiert werden. Bevorzugt liegt die Menge zwischen 0,1 und 10 Molprozent bezogen auf das Bernsteinsäureamid der Formel (VI), besonders bevorzugt zwischen 1 und 7 Molprozent bezogen auf das Bernsteinsäureamid der Formel (VI).

In einer weiteren Ausführungsform des Verfahrens ist es möglich, das nach der Isolierung des Produktes der allgemeinen Formel (I) durch Filtration erhaltene Filtrat, das bei der bevorzugten Verwendung eines Gemisches aus einem mit Wasser nicht oder nur wenig mischbaren organischen Verdünnungsmittels und Wasser zweiphasig ist, in dem nächsten Ansatz zur Durchführung des zweiten Schrittes des erfindungsgemäßen Verfahrens einzusetzen. Dies kann mehrfach wiederholt werden, bevorzugt bis zu zehnmal, besonders bevorzugt bis zu fünfmal. Dadurch wird nicht nur der Einsatz an Phasentransferkatalysator, bezogen auf Bernsteinsäureamid der allgemeinen Formel (VI), deutlich verringert, sondern gleichzeitig auch die benötigten Mengen an organischem Verdünnungsmittel und Wasser, was das Verfahren noch wirtschaftlicher macht.

Je nachdem, welcher Phasentransferkatalysator eingesetzt wird, ist es auch möglich, nur die organische Phase nach Abtrennung des gewünschten Produktes erneut einzusetzen.

Die Reaktionstemperatur im zweiten Schritt des erfindungsgemäßen Verfahrens kann in weiten Grenzen variiert werden und liegt zwischen 0°C und 200°C. Bevorzugt arbeiten man bei Temperaturen zwischen 20°C und 150°C, besonders bevorzugt zwischen 30°C und 130°C.

Die Reaktionszeit im zweiten Schritt des erfindungsgemäßen Verfahrens liegt zwischen 5 Minuten und 24 Stunden. Bevorzugt arbeitet man zwischen 30 Minuten und 12 Stunden, besonders bevorzugt zwischen 1 und 8 Stunden.

Das erfindungsgemäße Verfahren wird durch folgende Beispiele veranschaulicht, ohne auf sie eingeschränkt zu sein.

### Beispiel 1

Man legte 5,24 g [40 mmol] Bernsteinsäuremethylamid vor und tropfte unter Rühren bei 5°C 23,8 g [200 mmol] Thionylchlorid zu. Die resultierende Lösung wurde dann innerhalb von etwa 20 Minuten in 23,8 g [200 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde am Rotationsverdampfer eingeengt. Man erhielt 9,3 g eines dicken braunen Öles, das laut HPLC- und LC/MS-Analyse 11,1 Flächenprozent an Verbindung (VIII-1), 26,3 Flächenprozent an Verbindung (I-1), 5,8 Flächenprozent an Verbindung (IX-1, n=0) und 24,7 Flächenprozent an Verbindung (IX-1, n = 1) enthält.

Man löste dieses Öl in 30 ml Toluol, gab 0,2 g Aliquat^{®} 336 zu und tropft dann 10 ml Wasser zu. Dieses Gemisch wurde unter intensivem Rühren 4 Stunden auf 80°C erhitzt. Man ließ danach auf Raumtemperatur abkühlen, saugte den ausgefallenen Feststoff ab, wusch ihn mit Wasser und Ethanol, und trocknete. Man erhielt auf diese Weise 3,10 g schwarzen Feststoff, der laut HPLC-Analyse zu 99,3 Flächenprozent aus Verbindung (I-1) besteht, was einer Ausbeute von 54,5 % der Theorie entspricht.

### Beispiel 2

Man verfuhr wie in Beispiel 1, setzte jedoch 0,4 g einer 50%igen wässrigen Lösung von Benzyl-dodecyl-dimethylammoniumchlorid (Zephirol^{®}) als Phasentransferkatalysator zu. Man erhielt 3,27 g schwarzen Feststoff, der laut HPLC-Analyse zu 99,1 Flächenprozent aus Verbindung (I-1) besteht, was einer Ausbeute von 57,4 % der Theorie entspricht.

### Beispiel 3

Man verfuhr wie in Beispiel 1, setzte jedoch 0,2 g Dodecyl-trimethylammoniumbromid als Phasentransferkatalysator zu. Man erhielt 2,30 g schwarzen Feststoff, der laut HPLC-Analyse zu 99,0 Flächenprozent aus Verbindung (I-1) besteht, was einer Ausbeute von 40,3 % der Theorie entspricht.

### Beispiel 4

Man verfuhr wie in Beispiel 1, setzte jedoch 0,2 g Tetradecyl-trimethylammoniumbromid als Phasentransferkatalysator zu. Man erhielt 2,43 g schwarzen Feststoff, der lt. HPLC-Analyse zu 99,2 Flächenprozent aus Verbindung (I-1) besteht, was einer Ausbeute von 42,7 % der Theorie entspricht.

### Beispiel 5

Man verfuhr wie in Beispiel 1, setzte jedoch 0,2 g Tetraoctylammoniumbromid als Phasentransferkatalysator zu. Man erhielt 2,84 g schwarzen Feststoff, der lt. HPLC-Analyse zu 99,5 Flächenprozent aus Verbindung (I-1) besteht, was einer Ausbeute von 50,0 % der Theorie entspricht.

### Beispiel 6

Man verfuhr wie in Beispiel 1, setzte jedoch 0,4 g einer 50%igen wässrigen Lösung von Didecyldimethylammoniumchlorid als Phasentransferkatalysator zu. Man erhielt 3,45 g schwarzen Feststoff, der laut HPLC-Analyse zu 99,1 Flächenprozent aus Verbindung (I-1) besteht, was einer Ausbeute von 60,6 % der Theorie entspricht.

### Beispiel 7

Man legte 15,74 g [120 mmol] Bernsteinsäuremethylamid als Suspension in 49 ml Toluol vor und tropfte unter Rühren bei 10-20°C 24,9 g [209 mmol] Thionylchlorid zu. Man erhielt eine zweiphasige Lösung, von der man die untere Phase abtrennte und innerhalb von etwa 60 Minuten in 132,1 g [1110 mmol] Thionylchlorid, welches auf 60°C erwärmt wurde, eintropfte. Man erhitzte anschließend auf ca. 80°C und rührte 1 Stunde bei dieser Temperatur. Anschließend wurde das Reaktionsgemisch am Rotationsverdampfer eingeengt. Man erhielt 29,37 g eines dicken braunen Öls, das laut HPLC-Analyse 5,0 Flächenprozent an Verbindung (VIII-1), 14,5 Flächenprozent an Verbindung (I-1), 19,5 Flächenprozent an Verbindung (IX-1, n = 0) und 26,9 Flächenprozent an Verbindung (IX-1, n = 1) enthält.

Man löste dieses Öl in 90 ml Toluol, gab 0,6 g Aliquat^{®} 336 zu und tropfte dann 30 ml Wasser zu. Dieses Gemisch wurde unter intensivem Rühren 4 Stunden auf Rückfluss (85-88°C) erhitzt. Man ließ danach auf Raumtemperatur abkühlen, saugte den ausgefallenen Feststoff ab, wusch ihn mit 30 ml Wasser und zweimal je 30 ml Ethanol, und trocknete. Man erhielt auf diese Weise 11,10 g schwarzen Feststoff, der laut HPLC-Analyse zu 98,9 Flächenprozent aus Verbindung (I-1) besteht, was einer Ausbeute von 64,8 % der Theorie entspricht.

### Vergleichsbeispiel 1

Man legte 5,24 g [40 mmol] Bernsteinsäuremethylamid vor und tropft unter Rühren bei 5°C 23,8 g [200 mmol] Thionylchlorid zu. Die resultierende Lösung wurde dann innerhalb von etwa 20 Minuten in 23,8 g [200 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde am Rotationsverdampfer eingeengt. Man erhielt 10,3 g eines dicken braunen Öles, das laut HPLC- und LC/MS-Analyse 6,6 Flächenprozent an Verbindung (VIII-1), 19 Flächenprozent an Verbindung (I-1), 16,6 Flächenprozent an Verbindung (IX-1, n=0) und 34,1 Flächenprozent an Verbindung (IX-1, n = 1) enthält.

Man löste dieses Öl in 30 ml Toluol und tropft dann ohne Zugabe eines Phasentransferkatalysators 10 ml Wasser zu. Dieses Gemisch wurde unter intensivem Rühren 4 Stunden auf 80°C erhitzt. Man ließ danach auf Raumtemperatur abkühlen, saugte den ausgefallenen Feststoff ab, wusch ihn mit Wasser und Ethanol, und trocknete. Man erhielt auf diese Weise 0,90 g dunkelgrünen Feststoff, der laut HPLC-Analyse zu 98,6 Flächenprozent aus Verbindung (I-1) besteht, was einer Ausbeute von 15,7 % der Theorie entspricht.

### Beispiel 8

Man legte 5,24 g [40 mmol] Bernsteinsäuremethylamid vor und tropfte unter Rühren bei 5°C 23,8 g [200 mmol] Thionylchlorid zu. Die resultierende Lösung wurde dann innerhalb von etwa 20 Minuten in 23,8 g [200 mmol] Thionylchlorid, welches auf 60°C erwärmt wurde, eingetropft. Man erhitzte anschließend auf 80°C und rührt 1 Stunde bei dieser Temperatur. Das Reaktionsgemisch wurde am Rotationsverdampfer eingeengt. Man erhielt 9,7 g eines dicken braunen Öles, das laut HPLC-Analyse 4,2 Flächenprozent an Verbindung (VIII-1), 15,3 Flächenprozent an Verbindung (I-1), 20,1 Flächenprozent an Verbindung (IX-1, n = 0) und 28,3 Flächenprozent an Verbindung (IX-1, n = 1) enthält.

Man löste dieses Öl in 30 ml Chlorbenzol, gab 0,1 g Aliquat^{®} 336 zu und tropft dann 10 ml Wasser zu. Dieses Gemisch wurde unter intensivem Rühren 4 Stunden auf Rückfluss (ca. 94-97°C) erhitzt. Man ließ danach auf Raumtemperatur abkühlen, saugte den ausgefallenen Feststoff ab, wusch ihn mit Wasser und Ethanol, und trocknete. Man erhielt auf diese Weise 3,29 g schwarzen Feststoff, der laut HPLC-Analyse zu 99,4 Flächenprozent aus Verbindung (I-1) besteht, was einer Ausbeute von 57,9 % der Theorie entspricht.

### Beispiel 9

Man verfuhr wie in Beispiel 8, setzte jedoch 0,2 g von Tetraoctylammoniumbromid als Phasentransferkatalysator zu. Man erhielt 3,47 g schwarzen Feststoff, der laut HPLC-Analyse zu 98,7 Flächenprozent aus Verbindung (I-1) besteht, was einer Ausbeute von 60,7 % der Theorie entspricht.

### Vergleichsbeispiel 2

Man legte 5,24 g [40 mmol] Bernsteinsäuremethylamid vor und tropfte unter Rühren bei 5°C 23,8 g [200 mmol] Thionylchlorid zu. Die resultierende Lösung wurde dann innerhalb von etwa 20 Minuten in 23,8 g [200 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde am Rotationsverdampfer eingeengt. Man erhielt 9,5 g eines dicken braunen Öles, das laut HPLC-Analyse 7 Flächenprozent an Verbindung (VIII-1), 18,5 Flächenprozent an Verbindung (I-1), 17,6 Flächenprozent an Verbindung (IX-1, n = 0) und 30 Flächenprozent an Verbindung (IX-1, n = 1) enthält.

Man löste dieses Öl in 30 ml Chlorbenzol und tropfte dann ohne Zugabe eines Phasentransferkatalysators 10 ml Wasser zu. Dieses Gemisch wurde unter intensivem Rühren 4 Stunden auf 80°C erhitzt. Man ließ danach auf Raumtemperatur abkühlen, saugte den ausgefallenen Feststoff ab, wusch ihn mit Wasser und Ethanol, und trocknete. Man erhielt auf diese Weise 1,12 g schwarzen Feststoff, der laut HPLC-Analyse zu 98,8 Flächenprozent aus Verbindung (I-1) besteht, was einer Ausbeute von 19,6 % der Theorie entspricht.

### Beispiel 10

Man legte 26,23 g [200 mmol] Bernsteinsäuremethylamid in 35,3 g Toluol vor und tropfte unter Rühren bei 5-10°C 42,35 g [356 mmol] Thionylchlorid zu. Die bei Raumtemperatur resultierende Lösung wurde dann innerhalb von 2 Stunden in 225 g [1890 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde anschließend am Rotationsverdampfer eingeengt bis 70°C Badtemperatur und 25 mbar. Man erhielt als Rückstand 43,7 g Suspension. Man löste die noch warmen 43,7 g Rückstand in 174 g Toluol, gab 4,05 g Aliquat^{®} 336 zu und tropfte dann 55 ml Wasser innerhalb von etwa 5 Minuten, beginnend bei etwa 55°C, zu. Anschließend wurde unter intensivem Rühren 4 Stunden auf 80°C erhitzt. Man ließ danach auf Raumtemperatur abkühlen, saugte den ausgefallenen Feststoff ab, wusch ihn mit 55 ml Wasser und zweimal je 55 ml Ethanol, und trocknete. Man erhielt auf diese Weise 20,36 g schwarzen Feststoff, der laut HPLC-Analyse zu 98,6 Flächenprozent aus Verbindung (I-1) besteht, was einer Ausbeute von 71,1 % der Theorie entspricht.

### Beispiel 11

Man legte 8 g [61 mmol] Bernsteinsäuremethylamid in 13,4 g Chlorbenzol vor und tropfte unter Rühren bei 5-10°C 12,57 g [106 mmol] Thionylchlorid zu. Die bei Raumtemperatur resultierende Lösung wurde dann innerhalb von 1 Stunde in 66,6 g [560 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde anschließend am Rotationsverdampfer eingeengt. Man löste den noch warmen Rückstand in 79 g Toluol. Nach Zugabe von 2,4 g Aliquat^{®} 336 (ca. 9,7 Molprozent, bezogen auf Bernsteinsäuremethylamid) tropfte man 31 ml Wasser zu. Anschließend wurde unter intensivem Rühren 4 Stunden auf 75°C erhitzt. Man ließ danach auf Raumtemperatur abkühlen, saugte den ausgefallenen Feststoff ab , wusch ihn mit 30 ml Wasser und dann 30 ml Ethanol, und trocknete. Man erhielt auf diese Weise 6,4 g Feststoff, der laut HPLC-Analyse zu 98 Flächenprozent aus Verbindung (I-1) besteht, was einer Ausbeute von 73 % der Theorie entspricht, und 115 g zweiphasiges Filtrat.

### Beispiel 12

Man legte 8 g [61 mmol] Bernsteinsäuremethylamid in 13,4 g Chlorbenzol vor und tropfte unter Rühren bei 5-10°C 12,57 g [106 mmol] Thionylchlorid zu. Die bei Raumtemperatur resultierende Lösung wurde dann innerhalb von 1 Stunde in 66,6 g [560 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde anschließend am Rotationsverdampfer eingeengt. Zu dem noch warmen Rückstand wurde das Filtrat aus Beispiel 11 getropft. Anschließend wurde unter intensivem Rühren 4 Stunden auf 75°C erhitzt. Man ließ danach auf Raumtemperatur abkühlen, saugte den ausgefallenen Feststoff ab, wusch ihn mit 30 ml Wasser und dann 30 ml Ethanol, und trocknete. Man erhielt auf diese Weise 6,7 g Feststoff, der laut HPLC-Analyse zu 97,7 Flächenprozent aus Verbindung (I-1) besteht, was einer Ausbeute von 76 % der Theorie entspricht, und 114 g Filtrat.

### Beispiel 13

Man legte 8 g [61 mmol] Bernsteinsäuremethylamid in 13,4 g Chlorbenzol vor und tropfte unter Rühren bei 5-10°C 12,57 g [106 mmol] Thionylchlorid zu. Die bei Raumtemperatur resultierende Lösung wurde dann innerhalb von 1 Stunde in 66,6 g [560 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde anschließend am Rotationsverdampfer eingeengt. Zu dem noch warmen Rückstand wurde das Filtrat aus Beispiel 12 getropft. Anschließend wurde unter intensivem Rühren 4 Stunden auf 75°C erhitzt. Man ließ danach auf Raumtemperatur abkühlen, saugte den ausgefallenen Feststoff ab, wusch ihn mit 30 ml Wasser und dann 30 ml Ethanol, und trocknete. Man erhielt auf diese Weise 6,5 g Feststoff, der laut HPLC-Analyse zu 98,1 Flächenprozent aus Verbindung (I-1) besteht, was einer Ausbeute von 74 % der Theorie entspricht, und 119 g Filtrat.

### Beispiel 14

Man legte 8 g [61 mmol] Bernsteinsäuremethylamid in 13,4 g Chlorbenzol vor und tropfte unter Rühren bei 5-10°C 12,57 g [106 mmol] Thionylchlorid zu. Die bei Raumtemperatur resultierende Lösung wurde dann innerhalb von 1 Stunde in 66,6 g [560 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde anschließend am Rotationsverdampfer eingeengt. Zu dem noch warmen Rückstand wurde das Filtrat aus Beispiel 13 getropft. Anschließend wurde unter intensivem Rühren 4 Stunden auf 75°C erhitzt. Man ließ danach auf Raumtemperatur abkühlen, saugte den ausgefallenen Feststoff ab, wusch ihn mit 30 ml Wasser und dann 30 ml Ethanol, und trocknete. Man erhielt auf diese Weise 6,3 g Feststoff, der laut HPLC-Analyse zu 98 Flächenprozent aus Verbindung (I-1) besteht, was einer Ausbeute von 72 % der Theorie entspricht, und 114 g Filtrat.

### Beispiel 15

Man legte 8 g [61 mmol] Bernsteinsäuremethylamid in 13,4 g Chlorbenzol vor und tropfte unter Rühren bei 5-10°C 12,57 g [106 mmol] Thionylchlorid zu. Die bei Raumtemperatur resultierende Lösung wurde dann innerhalb von 1 Stunde in 66,6 g [560 mmol] Thionylchlorid, welches auf 60°C erwärmt wurde, eingetropft. Man erhitzte anschließend auf 80°C und rührte 1 Stunde bei dieser Temperatur. Das Reaktionsgemisch wurde anschließend am Rotationsverdampfer eingeengt. Zu dem noch warmen Rückstand wurde das Filtrat aus Beispiel 14 getropft. Anschließend wurde unter intensivem Rühren 4 Stunden auf 75°C erhitzt. Man ließ danach auf Raumtemperatur abkühlen, saugte den ausgefallenen Feststoff ab, wusch ihn mit 30 ml Wasser und dann 30 ml Ethanol, und trocknete. Man erhielt auf diese Weise 6,4 g Feststoff, der laut HPLC-Analyse zu 98,8 Flächenprozent aus Verbindung (I-1) besteht, was einer Ausbeute von 73 % der Theorie entspricht.

### Allgemeine Angaben:

HPLC-Bedingungen: Zorbax Eclipse Plus C18 4.6*50 mm 1.8µm, Eluent A: 0.1% H₃PO₄, Eluent B: Acetonitril, Gradient: 90/10, 20%/min, 5/95 (1.75), Fluss: 2 ml/min, 55°C.

## Patentansprüche

1. Verfahren zum Herstellen von Dithiin-tetracarboximiden der allgemeinen Formel (I) 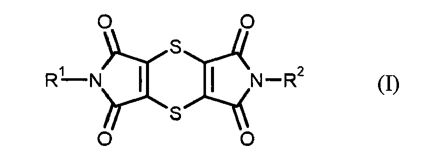 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
**dadurch gekennzeichnet, dass** man
in einer ersten Stufe Bernsteinsäuremonoamide der Formel (VI) 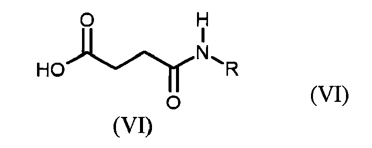 in welcher R für R¹ oder R² steht,
mit einem Überschuss an Thionylchlorid gegebenenfalls in Gegenwart eines Verdünnungsmittels umsetzt,
anschließend den Überschuss an Thionylchlorid entfernt und das so erhaltene Produktgemisch in einer zweiten Stufe in einem Gemisch aus einem organischen Lösungsmittel, Wasser und einem Phasentransferkatalysator in die Dithiin-tetracarboximide der Formel (I) überfuhrt

2. Verfahren gemäß Anspruch 1, **dadurch gekennzeichnet, dass** man im zweiten Schritt den Phasentransferkatalysator aus
(a) quaternären Ammoniumsalzen oder quaternären Phosphoniumsalzen der Formel (X) 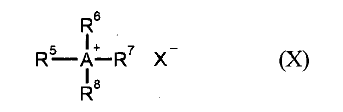 in welcher
R⁵, R⁶, R⁷ und R⁸ unabhängig voneinander gleich oder verschieden sind und für geradkettiges oder verzweigtes C₁-C₂₈-Alkyl, C₆-C₁₀-Aryl oder Benzyl stehen,
X für Halogen, Hydrogensulfat, Sulfat, Dihydrogenphosphat, Hydrogenphosphat, Phosphat oder Acetat (bevorzugt für Brom, Chlor, Fluor, Hydrogensulfat, Sulfat, Phosphat und Acetat) steht,
A für N oder P steht;
oder
(b) 4-Dialkylamino-pyridiniumsalzen oder Hexaalkyl-guanidiniumsalzen,
auswählt.

3. Verfahren gemäß Anspruch 1 oder 2, **dadurch gekennzeichnet, dass** man im zweiten Schritt einen Phasentransferkatalysator aus folgender Liste auswählt: Tetrabutylammoniumfluorid, -chlorid, -bromid, -iodid, -acetat, -hydrogensulfat, Tetraethylammoniumbromid, -iodid, Methyltributylammoniumchlorid, -bromid, -iodid, -acetat, -hydrogensulfat, Benzyl-dodecyl-dimethylammoniumchlorid, -bromid, Benzyltriethylammoniumbromid, -chlorid, Dodecyltrimethylammoniumchlorid, -bromid, Tetradecyl-trimethylammoniumchlorid, -bromid, Methyl-trioctylammoniumchlorid, Methyl-tridecylammoniumchlorid, Tetraoctylammoniumbromid, -chlorid, Didecyl-dimethyl-ammoniumchlorid, -bromid, Tetraphenylphosphoniumbromid, Ethyl-triphenylphosphoniumbromid, Ethyl-triphenylphosphoniumiodid und Ethyl-triphenylphosphoniumacetat.

4. Verfahren gemäß Anspruch 1, 2 oder 3, **dadurch gekennzeichnet, dass** man im zweiten Schritt organische Lösungsmittel einsetzt, welche nur geringfügig mit Wasser mischbar sind.

5. Verfahren gemäß Anspruch 1, 2, 3 oder 4, **dadurch gekennzeichnet, dass** man im zweiten Schritt Hexan, Heptan, Cyclohexan, Methylcyclohexan, Octan, Isooctan, Toluol, Xylole, Mesitylen, Ethylbenzol, Chlorbenzol, Dichlorbenzol, Nitrobenzol, Wasser oder Gemische dieser Verdünnungsmittel einsetzt.

6. Verfahren gemäß Anspruch 1, 2, 3, 4 oder 5, **dadurch gekennzeichnet, dass** man das nach der Isolierung des Produktes der allgemeinen Formel (I) durch Filtration erhaltene Filtrat, das bei der Verwendung eines Gemisches aus einem mit Wasser nicht oder nur wenig mischbaren organischen Verdünnungsmittels und Wasser zweiphasig ist, in dem nächsten Ansatz zur Durchführung des zweiten Schrittes des erfindungsgemäßen Verfahrens einsetzt.

7. Verfahren gemäß Anspruch 6, **dadurch gekennzeichnet, dass** das Filtrat bis zu zehnmal wieder verwendet wird.

8. Verfahren gemäß Anspruch 6 oder 7, **dadurch gekennzeichnet, dass** nur die organische Phase nach Abtrennung des gewünschten Produktes erneut eingesetzt wird.

## Claims

1. Process for preparing dithiine-tetracarboximides of the general formula (I) 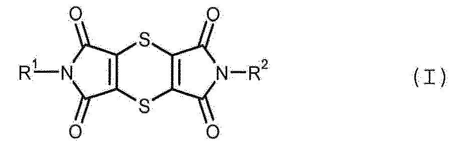 in which
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
**characterized in that**
in a first stage, succinic monoamides of the formula (VI) 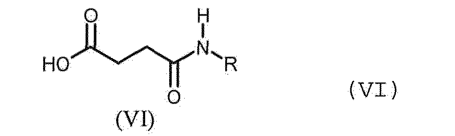 in which R is R¹ or R²
are reacted with an excess of thionyl chloride, optionally in the presence of a diluent,
then the excess of thionyl chloride is removed and the resulting product mixture is converted in a second stage, in a mixture of an organic solvent, water and a phase transfer catalyst, into the dithiine-tetracarboximides of the formula (I).

2. Process according to Claim 1, **characterized in that** in the second step the phase transfer catalyst is selected from
(a) quaternary ammonium salts or quaternary phosphonium salts of the formula (X) 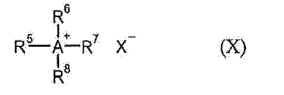 in which
R⁵, R⁶, R⁷ and R⁸ independently of one another are identical or different and are each straight-chain or branched C₁-C₂₈-alkyl, C₆-C₁₀-aryl or benzyl,
X is halogen, hydrogen sulphate, sulphate, dihydrogen phosphate, hydrogen phosphate, phosphate or acetate (preferably bromine, chlorine, fluorine, hydrogen sulphate, sulphate, phosphate and acetate),
A is N or P;
or
(b) 4-dialkylaminopyridinium salts or hexaalkylguanidinium salts .

3. Process according to Claim 1 or 2, **characterized in that** in the second step a phase transfer catalyst from the following list is selected: tetrabutylammonium fluoride, chloride, bromide, iodide, acetate and hydrogen sulphate, tetraethylammonium bromide and iodide, methyltributylammonium chloride, bromide, iodide, acetate and hydrogen sulphate, benzyldodecyldimethylammonium chloride and bromide, benzyltriethylammonium bromide and chloride, dodecyltrimethylammonium chloride and bromide, tetradecyltrimethylammonium chloride and bromide, methyltrioctylammonium chloride, methyltridecylammonium chloride, tetraoctylammonium bromide and chloride, didecyldimethylammonium chloride and bromide, tetraphenylphosphonium bromide, ethyltriphenylphosphonium bromide, ethyltriphenylphosphonium iodide and ethyltriphenylphosphonium acetate.

4. Process according to Claim 1, 2 or 3, **characterized in that** in the second step organic solvents are used which are only slightly miscible with water.

5. Process according to Claim 1, 2, 3 or 4, **characterized in that** hexane, heptane, cyclohexane, methylcyclohexane, octane, isooctane, toluene, xylenes, mesitylene, ethylbenzene, chlorobenzene, dichlorobenzene, nitrobenzene, water or mixtures of these diluents are used in the second step.

6. Process according to Claim 1, 2, 3, 4 or 5, **characterized in that** the filtrate which is obtained after the isolation of the product of the general formula (I) by filtration, and which is two-phase when a mixture is used of water and an organic diluent which is immiscible or only a little miscible with water, is employed in the next batch for carrying out the second step of the process of the invention.

7. Process according to Claim 6, **characterized in that** the filtrate is used again up to ten times.

8. Process according to Claim 6 or 7, **characterized in that** only the organic phase, following removal of the desired product, is re-used.

## Revendications

1. Procédé de fabrication dithiine-tétracarboximides de formule générale (I) 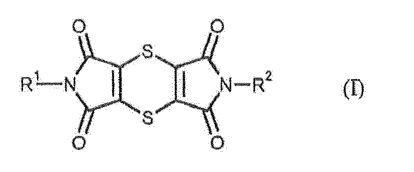 dans laquelle
R¹ et R² sont identiques ou différents et représentent hydrogène ; alkyle en C₁-C₈ éventuellement substitué une ou plusieurs fois par halogène, -OR³, -COR⁴ ; cycloalkyle en C₃-C₇ éventuellement substitué une ou plusieurs fois par halogène, allyle en C₁-C₄ ou halogénoalkyle en C₁-C₄ ; aryle ou aryl-(alkyle en C₁-C₄) chacun éventuellement substitué une ou plusieurs fois par halogène, alkyle en C₁-C₄, halogénoalkyle en C₁-C₄, - COR⁴ ou sulfonylamino,
R³ représente hydrogène, alkyle en C₁-C₄, alkylcarbonyle en C₁-C₄ ; ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
**caractérisé en ce que**
lors d'une première étape, des monoamides de l'acide succinique de formule (VI) 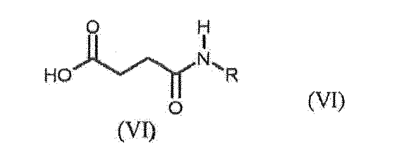
dans laquelle R représente R¹ ou R²,
sont mis en réaction avec un excès de chlorure de thionyle, éventuellement en présence d'un diluant,
puis l'excès de chlorure de thionyle est éliminé et le mélange de produits ainsi obtenu est transformé lors d'une seconde étape dans un mélange d'un solvant organique, d'eau et d'un catalyseur de transfert de phases en les dithiine-tétracarboximides de formule (I).

2. Procédé selon la revendication 1, **caractérisé en ce que**, lors de la seconde étape, le catalyseur de transfert de phases est choisi parmi
(a) les sels d'ammonium quaternaires ou les sels de phosphonium quaternaires de formule (X) 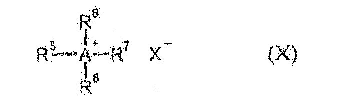 dans laquelle
R⁵, R⁶, R⁷ et R⁸ sont chacun indépendamment les uns des autres identiques ou différents et représentent alkyle en C₁-C₂₈ linéaire ou ramifié, aryle en C₆-C₁₀ ou benzyle,
X représente halogène, hydrogénosulfate, sulfate, dihydrogénosulfate, hydrogénophosphate, phosphate ou acétate (de préférence brome, chlore, fluor, hydrogénosulfate, sulfate, phosphate et acétate),
A représente N ou P ;
ou
(b) les sels de 4-dialkylamino-pyridinium ou les sels d'hexaalkyl-guanidinium.

3. Procédé selon la revendication 1 ou 2, **caractérisé en ce que**, lors de la seconde étape, un catalyseur de transfert de phases choisi dans la liste suivante est utilisé : fluorure, chlorure, bromure, iodure, acétate, hydrogénosulfate de tétrabutylammonium, bromure, iodure de tétraéthylammonium, chlorure, bromure, iodure, acétate, hydrogénosulfate de méthyltributylammonium, chlorure, bromure de benzyl-dodécyl-diméthylammonium, bromure, chlorure de benzyltriéthylammonium, chlorure, bromure de dodécyltriméthylammonium, chlorure, bromure de tétradécyl-triméthylammonium, chlorure de méthyl-trioctylammonium, chlorure de méthyl-tridécylammonium, bromure, chlorure de têtraoctylammonium, chlorure, bromure de didécyl-diméthyl-ammonium, bromure de tétraphénylphosphonium, bromure d'éthyl-triphénylphosphonium, iodure d'éthyl-triphénylphosphonium et acétate d'éthyl-triphénylphosphonium.

4. Procédé selon la revendication 1, 2 ou 3, **caractérisé en ce que** des solvants organiques qui ne sont miscibles que dans une faible mesure avec l'eau sont utilisés lors de la seconde étape.

5. Procédé selon la revendication 1, 2, 3 ou 4, **caractérisé en ce que** de l'hexane, de l'heptane, du cyclohexane, du méthylcyclohexane, de l'octane, de l'isooctane, du toluène, des xylènes, du mésitylène, de l'éthylbenzène, du chlorobenzène, du dichlorobenzène, du nitrobenzène, de l'eau ou des mélanges de ces diluants sont utilisés lors de la seconde étape.

6. Procédé selon la revendication 1, 2, 3, 4 ou 5, **caractérisé en ce que** le filtrat obtenu par filtration après l'isolement du produit de formule générale (I), qui est biphasé lors de l'utilisation d'un mélange d'un diluant organique non ou seulement peu miscible avec l'eau et d'eau, est utilisé dans la préparation suivante pour la réalisation de la seconde étape du procédé selon l'invention.

7. Procédé selon la revendication 6, **caractérisé en ce que** le filtrat est réutilisé jusqu'à dix fois.

8. Procédé selon la revendication 6 ou 7, **caractérisé en ce que** seule la phase organique est réutilisée après la séparation du produit souhaité.
